# EUROPÄISCHE PATENTSCHRIFT

(11) **EP 0 628 536 B1**
(45) Veröffentlichungstag und Bekanntmachung des Hinweises auf die Patenterteilung: **14.07.1999**
(21) Anmeldenummer: 94106896.7
(22) Anmeldetag: 03.05.1994
(51) Int. Cl.: C07C 209/62, C07C 209/58, C07C 211/52

(54) **Verfahren zur Herstellung von aromatischen Aminen**
Process for the preparation of aromatic amines
Procédé pour la préparation d'amines aromatiques

(30) Priorität: 11.05.1993 DE 4315623
(43) Veröffentlichungstag der Anmeldung: 14.12.1994
(73) Patentinhaber: Clariant GmbH, 65929 Frankfurt am Main (DE)
(72) Erfinder: Pfirmann, Ralf Dr., D-64347 Griesheim (DE)

(56) Entgegenhaltungen:
- EP-A- 0 367 010
- DE-A- 2 357 749
- DE-A- 2 826 439
- DATABASE WPI Week 8236 Derwent Publications Ltd., London, GB; AN 82-76025E XP002008443 & SU-A-878 764 (KHMELNITSKAYA I L) , 7.November 1981
- PATENT ABSTRACTS OF JAPAN vol. 11, no. 133 (C-418), 25.April 1987 & JP-A-61 271255 (IHARA CHEM IND CO LTD), 1.Dezember 1986,
- PATENT ABSTRACTS OF JAPAN vol. 007, no. 046 (C-153), 23.Februar 1983 & JP-A-57 200334 (SUMITOMO KAGAKU KOGYO KK), 8.Dezember 1982,
- ORGANIC REACTIONS, Nr. 3, 1946, Seiten 267-306, XP002008399 EVERETT S. WALLIS ET AL.: "The Hoffmann Reaction"

## Beschreibung

Die vorliegende Erfindung betrifft ein neues Verfahren zur Herstellung von Aminen durch eine vorteilhafte, neue Variante des Hofmann-Abbaus, wodurch Produkte, die bisher nur durch komplizierte Verfahren zugänglich gemacht werden konnten, einfach und in hohen Ausbeuten erhalten werden können.

Das Verfahren dient zur Herstellung von aromatischen Aminen, insbesondere von Anilinen, aus den entsprechenden Amiden, insbesondere den Benzamiden, wobei diese Amine wichtige Vorprodukte für die Herstellung von Flüssigkristallen, Pflanzenschutzmitteln und Pharmaka darstellen. Beispielsweise können nach dem erfindungsgemäßen Verfahren 2,6-Dihaloaniline, insbesondere 2,6-Difluoranilin und 2,6-Dichloranilin, hergestellt werden. Des weiteren ist es möglich, 4-Trifluormethylanilin herzustellen. An diesen Beispielen soll kurz die Verwendung der herstellbaren Amine und die Herkunft der Ausgangsmaterialien erläutert werden.

2,6-Difluoranilin dient zum Beispiel als Zwischenprodukt für die Herstellung von Pharmaka (EOS 497 564, PCT WO 9115464) und Flüssigkristallen für die Anwendung in Displays (JP 04029976). 2,6-Dichloranilin kann u.a. zu hochwirksamen Pharmaka (EOS 497 564, US 5130441, A. Andreani et al., Acta Pharm. Nord., 4 (2), 93-96) verarbeitet werden. 4-Trifluormethylanilin dient neben anderen Anwendungen zur Herstellung von Anthelmintika (US 5034410) und entzündungshemmenden und immunmodulierenden Mitteln (US 5001124). Die erfindungsgemäß herstellbaren Vorprodukte können in die Wirkstoffe oder die aktiven Verbindungen nach den in dcr zitierten Literatur angegebenen Methoden überführt werden.

Die DE-A-23 57 749 betrifft ein Verfahren zur Herstellung von Aminen durch Umsetzung von Carbonsäureamiden mit Hypochloriten in Gegenwart von überschüssigem Alkalihydroxid und von Polymerisationsinhibitoren.

Die DE-A-28 26 439 beschreibt ein Verfahren zur Herstellung von Aminen, wobei ein Amid bei pH ≤ 7 zunächst mit gasförmigem Chlor in Anwesenheit oder Abwesenheit eines Verdünnungsmittels und das gebildete N-Chloramid nachfolgend mit einem Alkali- oder Erdalkalihydroxid umgestzt wird.

Gemäß SU-878 764 lassen sich durch Hofmann Abbau Anilin, o-, m- oder p-Nitroanilin in größerer Reinheit und Ausbeute herstellen, indem man zu dem Reaktionsgemisch allmählich NaOCl bei 5 bis 22 °C zugibt und die Temperatur während der Zugabe konstant hält.

2,6-Difluorbenzamid, Ausgangsmaterial für 2,6-Difluoranilin, wird durch allgemein in der Literatur bekannte Methoden aus 2,6-Difluorbenzonitril gewonnen (J. March, Advanced Organic Chemistry (1985), 788). Möglich ist zum Beispiel die Umsetzung von 2,6-Difluorbenzonitril in wäßrig-alkalischem Medium mit Wasserstoffperoxid (JP-OS 60-132 942). Auf analoge Weise gelingt die Herstellung von 2,6-Dichlorbenzamid aus 2,6-Dichlorbenzonitril. 4-Trifluormethylbenzamid kann zum Beispiel aus 4-Trifluormethylbenzonitril (J.X. Wang et al., J. Chem. Res., Synop., (12), 456-457) oder aus 4-Trifluormethylbenzoesäure (D.E. Walch et al., J. Med. Chem. 12 (1969), 299-303) hergestellt werden.

Hofmann-Abbau-Reaktionen, wie die inredestehenden verlaufen, besonders bei 2,6-disubstituierten und/oder elektronenarmen Amiden, nur mit mäßigen Ausbeuten und Selektivitäten. Dabei treten unter den drastischen Umsetzungsbedingungen als häufige Nebenreaktionen Hydrolyse des Amides , Ringchlorierung und oxidativer Abbau, besonders durch Oxidation am Stickstoff, verursacht durch die notwendigen hohen Reaktionstemperaturen, auf. In einigen Fällen wird ein Zusatz von teuren oder toxischen Hilfsstoffen wie zum Beispiel Phasentransferkatalysatoren notwendig (JP 61 271255). Durch diese negativen Einflüsse wird die Herstellung dieser Amine in vielen Fällen unwirtschaftlich.

Bei 2,6-disubstituierten Amiden ist die Addition von Hydroxid an das intermediäre Isocyanat aus sterischen Gründen ungünstig, besonders wenn größere Substituenten anwesend sind (Org. React. 3, 277-282 (1946)). Solche Umsetzungen sind daher auch kaum bekannt.

Weiterhin wird durch elektronenziehende Substituenten die Hydrolyse der Amidbindung im Edukts zum Carboxylat erleichtert (unerwünscht), wobei gleichzeitig die Umlagerung verlangsamt wird.
Bei elektronenabgebenden Substituenten wie Alkoxy- oder Hydroxy-Gruppen ist die Umlagerung begünstigt, jedoch wird unvorteilhafterweise die Ringchlorierung ebenfalls beschleunigt. ( z.B. Haufer et al., J. Am. Chem. Soc. 59, 121 (1937) ibid. 60, 2308 (1937), ibid. 61, 618 (1939)).
Bekannt ist außerdem, daß Temperaturerhöhung zwar die Umlagerung gegenüber unerwünschter Hydrolyse begünstigt, jedoch kommt hierdurch der stark oxidierende und halogenierende Charakter des Reagenzes wieder zum Tragen. Dies ist aber besonders für oxidationsempfindliche Amine, insbesondere für aromatische Amine wenig vorteilhaft und nicht erwünscht.

Daher bestand ein sehr hoher Bedarf nach einem neuen Verfahren zur Herstellung von aromatischen Aminen, das die beschriebenen Nachteile nicht aufweist, von leicht zugänglichen Stoffen ausgeht, die gewünschten Verbindungen in hoher Ausbeute zugänglich macht und sich zudem technisch ohne großen Aufwand realisieren läßt.

Gelöst wird diese Aufgabe durch ein Verfahren zur Herstellung von aromatischen Aminen durch Umsetzung von aromatischen Amiden in wäßrig-alkalischen Lösungen und/oder Suspensionen mit Halogenen oder Hypohalogeniten durch Hofmann-Abbau-Reaktion. Es ist dadurch gekennzeichnet, daß man in Gegenwart von (C₁-C₈)-Alkylgruppen, oder Benzylalkoholen bei -15 bis 80 °C umsetzt und die Reaktionsprodukte durch Hydrolyse, Hydrierung oder reduktive Methoden in Amine überführt.

Durch dieses neue Verfahren lassen sich überraschenderweise viele aromatische Amide in die entsprechenden Amine überführen.

Der aromatische Rest kann Phenyl, Naphthyl oder Heteroaryl wie z.B. Pyridin, Thiophen oder Pyrrol sein. Der aromatische Rest kann auch durch eine beliebige Anzahl Chlor-, Fluor- oder Bromatome, Trifluormethyl-, Nitro-, Cyano-, Carboxyl-, (C₁-C₄)-Alkoxycarbonyl-, (C₁-C₄)-Alkyl oder (C₁-C₄)-Alkoxygruppen substituiert sein.

Die aromatischen Amide mit Chlor, Brom, Natriumhypochlorit oder Natriumhypobromit in wäßrig alkalischem Medium in Gegenwart von Alkoholen umgesetzt.

Man kann unter Verwendung von Hypohalogenit-Lösungen (Bleichlaugen) arbeiten, was der Dosierung von elementarem Halogen zu alkalisch wirkenden Lösungen äquivalent ist. Zur Beschreibung der Reaktionsbedingungen ist die Angabe der eingesetzten Halogenmenge ausreichend, da sich in situ bei Kontakt von Halogen mit den eingesetzten alkalisch wirkenden wäßrigen Lösungen Hypohalogenitlösungen bilden. Man verwendet daher Chlor oder Brom in Mengen zwischen etwa 1 Mol bis etwa 5 Mol, insbesondere etwa 1.01 Mol bis etwa 2 Mol, besonders bevorzugt zwischen etwa 1.02 Mol bis etwa 1.2 Mol, jeweils bezogen auf 1 Mol abzubauendes Amid. Die Verwendung von Chlor ist wegen der besseren technischen Verfügbarkeit bevorzugt. Das Halogen kann zugetropft (Brom) oder gasförmig eingeleitet (Chlor) werden. Je nach Reaktionstemperatur und Größe des Ansatzes kommen dabei Dosierzeiten zwischen etwa 0.5 h bis etwa 16 h, bevorzugt etwa 1 h bis 4 h in Frage, wobei die vorstehende Einschränkung hinsichtlich der Dosierzeit gilt, die durch die notwendige Entfernung der bei der exotherm verlaufenden Reaktion gebildeten Wärme bedingt wird.

Dosiert man Bleichlaugen, also wäßrige Hyphalogenit-Lösungen, was im allgemeinen im Labormaßstab gegenüber elementaren Halogen Handhabungsvorteile aufweist, so verwendet man Lösungen mit einem Gehalt an aktivem Chlor von etwa 30 bis etwa 250 g pro kg Lösung, bevorzugt zwischen etwa 100 und etwa 160 g aktiven Chlor pro kg Lösung bzw. etwa 60 bis etwa 550 g aktivem Brom pro kg Lösung, bevorzugt zwischen etwa 200 bis 350 g pro kg Lösung. Diese Lösungen können durch Dosierungen der entsprechenden Mengen Chlor oder Brom in wäßrige, alkalisch wirkende Lösungen erhalten werden.

Man arbeitet bei der Umsetzung im Sinne des Hofmann-Abbaus üblicherweise bei Temperaturen zwischen etwa -15 °C und etwa 80 °C, bevorzugt zwischen etwa 0 °C und etwa 50 °C, besonders bevorzugt zwischen etwa 10 °C und etwa 40 °C.

Diese niedrigen Temperaturen sind besonders vorteilhaft, da durch die niedrige Temperatur die Oxidationswirkung des Chlors, die bei herkömmlichen Verfahren zu vielen Nebenprodukten führt, stark verringert wird.

Die Abbaureaktion der aromatischen Amide zu den Aminen wird erfindungsgemäß in Gegenwart von Alkoholen durchgeführt. Als Alkohole können hierbei-(C₁-C₈)-Alkanole jeder Konstitution, die zusätzlich durch Alkoxy(C₁-C₄)gruppen, Fluor-, Chlor- oder Bromatome, Nitrogruppen, Cyanogruppen, Trifluormethylgruppen oder Alkoxy(C₁-C₄)-carbonylgruppen substituiert sein können oder Phenylmethanole, wobei der Phenylrest durch Alkyl(C₁-C₄)gruppen, Alkoxy(C₁-C₄)gruppen, Fluor-, Chlor- oder Bromatome, Nitrogruppen, Cyanogruppen, Trifluormethylgruppen, Alkoxy-(C₁-C₄)-carbonylgruppen substituiert sein kann, verwendet werden. Besonders geeignet sind primäre Alkohole, insbesondere Methanol, Ethanol und Benzylalkohol.

In vielen Fällen hat es sich bewährt, die Alkohole in Mengen von 1 bis 20 Mol, insbesondere 1,1 bis 10 Mol, bevorzugt 1,05 bis 5 Mol pro Mol Amid einzusetzen.

Die Alkohole werden in Mischungen mit alkalisch wirkenden Lösungen oder Suspensionen eingesetzt. Diese können aus Alkali- oder Erdalkalimetallverbindungen hergestellt werden. Solche alkalisch wirkende Verbindungen sind zum Beispiel Hydroxide, Carbonate, Hydrogencarbonate, Phosphate, Hydrogenphosphate, Dihydrogenphosphate, Oxide oder ähnliche Verbindungen oder Mischungen derselben, insbesondere die entsprechenden Alkalimetallverbindungen bevorzugt Natriumhydroxid, Kaliumhydroxid, Natriumcarbonat und/oder Kaliumcarbonat. Man verwendet die alkalisch wirkenden Verbindungen in Mengen, bezogen auf abzubauendes Amid, zwischen etwa 1 Mol bis etwa 30 Mol, bevorzugt zwischen etwa 3 Mol bis etwa 15 Mol, besonders bevorzugt zwischen etwa 5 Mol und etwa 10 Mol. Die Konzentrationen der wäßrigen Lösungen hängen vom eingesetzten Amid ab, betragen jedoch typischerweise zwischen etwa 1 Mol/l bis etwa 20 Mol/l, bevorzugt zwischen 3 Mol/l und 10 Mol/l. Die Menge der alkalisch wirkenden wäßrigen Suspension wird in praxi so gewählt, daß die Rührbarkeit der Reaktionmischung gewährleistet bleibt. Die Reaktionszeiten betragen etwa 0.5 h bis etwa 16 h, je nach Geschwindigkeit der Dosierung, die aufgrund des Verlaufs der Umsetzungen von den verfügbaren Kühlflächen abhängen kann. Das primäre Reaktionsprodukt aus der im Sinne eines Hofmann-Abbaus ablaufenden, erfindungsgemäßen Umsetzung ist vermutlich ein Alkylcarbamat des gewünschten Amins, da wahrscheinlich das als Zwischenstufe auftretende Isocyanat durch die unter Reaktionsbedingungen nucleophilen Alkohole abgefangen wird. Dadurch werden die oxidationsempfindlichen Amine überraschenderweise der oxidierenden Einwirkung der Halogene entzogen, und es gelingt somit, die Amine erst nach dem Umlagerungsschritt durch Hydrolyse, Hydrierung oder allgemein durch Reduktion gezielt freizusetzen.

Es ist bekannt, Amine, insbesondere Aniline, aus Carbamaten zu gewinnen. (T.W. Greene, P.G.M. Wuts, Protective Groups in Organic Chemistry (1991), 317-348). Im vorliegenden Fall gelingt die Abspaltung der Aminogruppe durch einfaches Erhitzen der erfindungsgemäßen Reaktionsmischung, nachdem ein eventueller Chlor- oder Bromüberschuß vernichtet worden ist. Gegebenenfalls substituierte Benzylcarbamate lassen sich unter alkalischen Bedingungen bei 80 bis 100 °C in weniger als 10 h hydrolysieren, während einfache Alkylcarbamate bei denselben Temperaturen Reaktionszeiten bis zu 96 h benötigen.

Die Reaktionsprodukte können im allgemeinen einfach durch Phasentrennung isoliert werden, wobei Mischungen mit den eingesetzten Alkoholen anfallen. Die Phasentrennung kann durch Zusatz von zusätzlichen Lösungsmitteln wie zum Beispiel Toluol oder Xylol verbessert oder herbeigeführt werden. Der abgespaltene Alkohol kann gegebenenfalls nach Abtrennung und Reinigung, wieder in das erfindungsgemäße Verfahren eingesetzt werden.

Besonders günstig ist die Verwendung von gegebenenfalls substituiertem Benzylalkohol, da sich die entsprechenden Benzylcarbamate ohne großen Aufwand und daher kostengünstig auch durch Hydrierung spalten lassen (T.W. Greene et al., loc. cit., 335-341).

Besonders einfach gestaltet sich die Hydrierung unter Verwendung von Wasserstoff-Gas in Anwesenheit von Übergangsmetallkatalysatoren, insbesondere von Palladium auf Aktivkohle. Neben Palladium kommen als eingesetzte Übergangsmetalle insbesondere Nickel oder Platin in Frage. Die Hydrierungen verlaufen glatt unter Wasserstoffdruck zwischen etwa 1.1 bar und etwa 100 bar bei Temperaturen zwischen etwa 10 °C und etwa 80 °C in niederen aliphatischen Alkoholen oder einfachen aromatischen oder aliphatischen Kohlenwasserstoffen wie zum Beispiel Hexan, Methylcyclohexan, Toluol, Xylol, Methanol, Butanol, Isopropanol oder Ethanol als Lösungsmittel oder Mischungen derer. Die Edelmetallkatalysatoren werden in Mengen zwischen etwa 0.05 bis etwa 3, bevorzugt etwa 0.3 bis 1 Massen-% (als reines Übergangsmetall gerechnet) eingesetzt.

Alternativ möglich ist die Verwendung der sogenannten Transfer-Hydrierung zur Synthese der neuen Zwischenprodukte (T.W. Greene et al., loc. cit., 156-160).

Die Konzentration des Endproduktes in der Mutterlauge der Hydrierung beträgt typischerweise zwischen etwa 10 bis 500, bevorzugt zwischen etwa 100 bis 300 g/l. Setzt man dabei die durch Umsetzung mit unsubstituiertem Benzylalkohol erhaltenen Reaktionsprodukte ein, so erhält man bei der Hydrierung neben dem gewünschten Amin noch Toluol. Daher ist Toluol als Lösungsmittel für die Hydrierung bevorzugt. Das Endprodukt kann im allgemeinen durch Einengen der vom Katalysator gegebenenfalls heiß filtrierten Lösungen und anschließende Filtration gewonnen werden. Dabei bietet es sich an, unter Anwesenheit von oxidationshindernden Zusätzen wie zum Beispiel Hydrazin oder Hydraziniumsalzen oder 2,6-Di-tert.butyl-4-methylphenol zu arbeiten, da insbesondere die durch das erfindungsgemäße Verfahren hergestellten Aniline als Endprodukte hohe Labilität gegen Luftsauerstoff, besonders beim Erwärmen zeigen.

Neben Hydrolyse und Hydrierung können auch allgemein reduktive Methoden zum Freisetzen der Amins angewendet werden. Unter reduktiven Methoden werden die Umsetzung des Reaktionsproduktes mit Organoaluminiumhydriden, Borhydriden, Siliciumhydriden, Metallen und/oder Hydrazin verstanden.

Das gewünschte Wertprodukt läßt sich je nach Stoffeigenschaften (Aggregatzustand, Lösungsverhalten) durch die üblichen Isolierungs- und Reinigungsmethoden erhalten. Bei flüssigen Produkten bietet sich besonders eine einfache Phasentrennung an, die durch Zusätze von Lösungsmitteln verbessert werden kann. Viele der herzustellenden Amine sind mit Wasserdampf flüchtig, sodaß für die Abtrennung des Produktes aus dem Reaktionsgemisch die Wasserdampfdestillation eine schonende und technisch leicht zu verwirklichende Methode darstellt. Feste Produkte können durch Filtration oder Extraktion isoliert und anschließend durch Kristallisation gereinigt werden. Für flüssige und feste Produkte kann sowohl Destillation als auch Chromatographie zur Reinigung angewandt werden, flüssige Produkte werden in der Regel fraktioniert.

In einer besonders bevorzugten Ausführungsform des Verfahrens wird 2,6-Difluorbenzamid zu 2,6-Difluoranilin umgesetzt.

Die folgenden Beispiele erläutern das Verfahren ohne es zu beschränken.

### Beispiel 1

In 450 g Wasser werden 180 g Benzylalkohol und 269 g (6.725 Mol) Natriumhydroxid eingetragen. Bei 20 °C gibt man 220 g (0.9 Mol) 2-Benzyloxy-6-fluorbenzamid zu und leitet bei 40 °C unter guter Rührung Chlor ein (15 l/h). Die Umsetzung wird gaschromatographisch überwacht und bei vollständigem Umsatz nach 2.5 h abgebrochen.

(Durch Extraktion mit Methyl-tert.-butylether (MTBE), Trocknen über Magnesiumsulfat und Entfernen des Lösungsmittels kann an dieser Stelle 2-Benzyloxy-6-fluoranilin als leicht braun gefärbtes, zähes Öl gewonnen werden, sofern man nach dem Vernichten überschüssigen Chlors die Mischung 3 h auf 95 °C erhitzt (siehe auch Beispiel 3, auf diesen Schritt wird jedoch hier verzichtet).

### Verfahrensweise a

Man heizt die wäßrige Mutterlauge 3 h auf 95 °C, nachdem ein eventueller Überschuß Chlor durch Zugabe von Natriumsulfit zerstört worden ist. Die organische Phase wird von der wäßrigen Phase getrennt, in 200 ml Methanol aufgenommen und danach mit 5 g Pd/C (5 % Pd, 50 %ig feucht) solange unter H₂-Atmosphäre (schwacher Überdruck) kräftig gerührt (15 h), bis keine Verbindungen mit Benzyloxygruppierungen mehr nachzuweisen sind. Man filtriert den Katalysator ab und wäscht mit Methanol nach. Unter Inertgas wird das Methanol im wesentlichen abdestilliert und 300 g Toluol zugegeben. Nach dem Kühlen (0 °C) erhält man 60.9 g (0.48 Mol, 53 %) nach dem Trocknen hellbraun bis dunkelgrau gefärbtes 2-Amino-3-fluorphenol erhalten (Gehalt (GC): 100 %). In der schwarzen Mutterlauge sind weitere 24.2 g (0.19 Mol, 21 %) Produkt enthalten, wie durch quantitative Gaschromatographie ermittelt wird. Die Mutterlauge wird bei weiteren Ansätzen wieder verwendet.

### Verfahrensweise b

Nach einigen Minuten ohne Rührung werden die Phasen getrennt, die organische Phase wird mit 300 g Methanol versetzt und mittels 5 g Pd/C (5 % Pd, 50 %ig feucht) unter H₂-Atmosphäre (schwacher Überdruck) kräftig gerührt (20 h). Die GC-Analyse weist Lösungsmittel und neben untergeordneten Mengen von Nebenprodukten 2-Amino-3-fluorphenol als Hauptkomponente aus. Man filtriert den Katalysator ab und wäscht mit Methanol nach. Unter Inertgas wird das Methanol aus dem Filtrat im wesentlichen abdestilliert und 300 g Toluol zugegeben. Nach dem Kühlen (0 °C) und nach Trocknen erhält man 65.3 g (0.51 Mol, 57 %) hellbraun gefärbtes 2-Amino-3-fluorphenol. (Gehalt (GC): 100 %). In der Mutterlauge sind weitere 28.2 g (0.22 Mol, 25 %) Produkt enthalten, wie durch quantitative Gaschromatographie ermittelt wird. Die Mutterlauge wird bei weiteren Ansätzen wieder verwendet.

### Beispiel 2

In 140 g Wasser werden 60 g (1.5 Mol) Natriumhydroxid, 40 g (0.37 Mol) Benzylalkohol und 25.8 g (0.164 Mol) 2,6-Difluorbenzamid vorgelegt. Bei 20-30°C (leichte Exothermie) wird in diese Suspension ein Chlorstrom von 5 l/h eingeleitet. Nach 1 h ist die Umsetzung beendet, die Dosierung von Chlor wird beendet und in die inzwischen orangefarbene, klare Lösung wird bei 100 °C Wasserdampf eingeleitet. Nach 6 h geht kein 2,6-Difluoranilin mehr über, die zwei Phasen der Mutterlauge werden getrennt, die Extraktion der wäßrigen Phase (MTBE) ergibt nur noch einen vernachlässigbaren Gehalt an 2,6-Difluoranilin. Die organischen Phasen werden vereinigt (88.2 g), quantitative Analyse ergab einen Gehalt an 2,6-Difluoranilin von 19.4 g (0.15 Mol, 92 %). Durch Fraktionierung (Normaldruck) kann das Produkt rein erhalten werden.

### Beispiel 3

Es werden 120 g Methanol, 70 g Wasser, 30 g (0.75 Mol) Natriumhydroxid und 24.5 g (0.1 Mol) 2-Benzyloxy-6-fluorbenzamid vorgelegt und auf 40 °C erhitzt. Man leitet Chlor ein (4 l/h), wobei sich die farblose Suspension nach kurzer Zeit bräunlich verfärbt und die Heizung abgeschaltet werden kann, da sich die Temperatur wegen des exothermen Verlaufs der Umsetzung hält. Nach 25 min ist die Umsetzung beendet, wie durch GC nachgewiesen werden kann. Statt der anfänglich eingesetzten Suspension wird eine klare Lösung erhalten. Man destilliert das Methanol im schwachen Vakuum ab (50 °C) und erhitzt die erhaltene Suspension von 2-Benzyloxy-6-fluor-N-carboxymethoxy-anilin 48 h auf 100 °C. Nach dem Abkühlen werden 50 g Toluol zugegeben, die Phasen getrennt und die organische Phase mit 2 g MgSO₄ und 1 g Aktivkohle versetzt und einige Stunden gerührt. Nach Filtration und Entfernen des Lösungsmittels am Rotationsverdampfer erhält man 19.8 g (91 mMol, 91 %) 2-Benzyloxy-6-fluoranilin als bräunliches, klares Öl, das für weitere Umsetzungen eine ausgezeichnete Reinheit (GC: > 96 %) aufweist.

2-Benzyloxy-6-fluor-N-carbomethoxy-anilin kann isoliert werden, wenn man wie in Beispiel 1 bereits angegeben verfährt, jedoch auf die 48-stündige Hydrolyse des Zwischenproduktes verzichtet und dieses nach üblichen Methoden (insbesondere Filtration und Umkristallisation) isoliert und reinigt.

### Beispiel 4 (Vergleichsbeispiel nach literaturbekannten Methoden)

Man legt 110 g Wasser, 109.4 g (1.368 Mol) 50 %ige Natronlauge und 86.4 g (0.547 Mol) 2,6-Difluorbenzamid vor und dosiert bei 65 °C in 1 h 262.5 g (0.602 Mol) 17 %ige Chlorbleichlauge zu. Durch den exothermen Verlauf der Umsetzung ist es möglich, die Heizung abzuschalten. Nach weiteren 30 min bei 70 °C heizt man auf 100 °C und leitet Wasserdampf ein. Nachdem kein Produkt mehr übergeht, trennt man die organische Phase im Destillat ab, extrahiert die wäßrige Phase mit n-Hexan (30 g) nach und destilliert die vereinigten und über Magnesiumsulfat getrockneten organischen Phasen bei Normaldruck über eine kurze Vigreux-Kolonne. Bei 154-156 °C gehen 33.5 g (0.26 Mol, 47 %) 2,6-Difluoranilin als leicht gelbliche Flüssigkeit über.

### Beispiel 5

In 1000 g Wasser und 250 g Ethanol werden 190.0 g (1 Mol) 2,6-Dichlorbenzamid mit 336.7 g (6 Mol) Kaliumhydroxid vermischt und die Suspension auf 50 °C erhitzt. Man leitet bei 55 °C 4 h Chlor ein (6 l/h), zerstört mittels Natriumsulfit überschüssiges Halogen und erhitzt danach 36 h auf 99 °C. Danach leitet man in die Mischung bei 100 °C Wasserdampf ein und destilliert das Produkt ab. Die Destillate werden bei 0-10 °C kaltgerührt und anschließend 2,6-Dichloranilin abgesaugt. Nach dem Trocknen erhält man 147.3 (0.909 Mol, 91 %) 2,6-Dichloranilin als farblosen Festkörper.

### Beispiel 6

Man trägt in eine Suspension von 296.4 g (4 Mol) Calciumhydroxid und 80 g Natriumhydroxid in 800 g Wasser 189.1 g (1 Mol) 4-Trifluormethylbenzamid und 108 g (1 Mol) Benzylalkohol ein und dosiert anschließend bei 20 °C binnen 6 h 520 g (1.04 Mol) 14.8 %ige Chlorbleichlauge zu. Man rührt bei 35 °C 6 h nach und gibt dann zu der Lösung bei 20 °C 50 g Toluol zu. Man trennt die Phasen und setzt der organischen Phase 300 g Methanol zu. Mittels 3 g Palladium auf Aktivkohle (5 % Pd, 50 %ig feucht) wird die Mischung unter schwachem Wasserstoffdruck bei 40 °C unter kräftigem Rühren 20 h hydriert. Von der verbleibenden Mischung filtriert man den Katalysator ab und destilliert die Lösungsmittel über. Der verbleibende Rückstand wird destilliert, bei 12 Torr/81-86 °C geht leicht gelbliches 4-Trifluormethylanilin über. Man erhält 129.1 (0.801 Mol, 80 %) Trifluormethylanilin.

### Beispiel 7

In 232,3 g (2,15 Mol) Benzylalkohol werden 261,2 g (1,66 Mol) 2,6-Difluorbenzamid suspendiert, die Suspension wird auf 55 °C geheizt und in 2 h werden 233,3 g (2,08 Mol) 50 %ige Kaliumhydroxid-Lösung zugetropft. Nach dieser Zeit wird die Temperatur auf 60 °C erhöht und 3 h weitergerüht. Anschließend gibt man bei 10 °C weitere 600 g Wasser, 280 g (7 Mol) Natriumhydroxid und 185 g (1.71 Mol) Benzylalkohol zu und leitet bei 40 °C 3 h Chlor ein (15-18 l/h). Die Phasen werden getrennt, die organische Phase wird mit 500 ml Methanol und 7 g Pd/C (5 % Pd, 50 % feucht) versetzt und wie in Beispiel 1b beschrieben, hydriert. Nach Aufarbeitung analog zu Beispiel 1b erhält man 123.5 g (0.97 Mol, 59 %) 2-Amino-3-fluorphenol als hellgraue Flitter.

### Beispiel 8

### 2-Amino-3-fluorphenol ausgehend von 2,6-Difluorbenzonitril im Eintopfverfahren hergestellt:

In 200 ml Benzylalkohol werden 69.9 g (0.5 Mol) 2,6-Difluorbenzonitril und 237 g (1.2 Mol) 6-normale Natronlauge vorgelegt. Dazu tropft man 221 g (1.95 Mol) 30 %ige Wasserstoffperoxid-Lösung binnen 30 min, wobei die Temperatur zu Beginn der Dosierung von 20 °C auf 50 °C ansteigt und auf diesen Wert gehalten wird. Nach 5 h (vollständiger Umsatz zu 2-Benzyloxy-6-fluorbenzamid kann mittels GC nachgewiesen werden) wird abgekühlt und mit 200 g Wasser und 60 g (1.5 Mol) Natriumhydroxid ergänzt. Man leitet 2 h bei 30 °C Chlor ein (8-10 l/h), die Umsetzung wird gaschromatographisch verfolgt und bei Verschwinden des Amides abgebrochen. Man trennt die Phasen, versetzt die organische Phase mit 180 g Methanol und arbeitet weiter, wie in Beispiel 7 angegeben. Man erhält 32.4 g (0.255 Mol, 51 %) 2-Amino-3-fluorphenol als braunschwarzes Pulver.

## Patentansprüche

1. Verfahren zur Herstellung von aromatischen Aminen durch Umsetzung von aromatischen Amiden in wäßrig-alkalischen Lösungen und/oder Suspensionen mit Halogenen oder Hypohalogeniten durch Hofmann-Abbau-Reaktion, dadurch gekennzeichnet, daß man in Gegenwart von (C₁-C₈)-Alkanolen oder Benzylalkoholen bei -15 bis 80 °C umsetzt und die Reaktionsprodukte durch Hydrolyse, Hydrierung oder reduktive Methoden in Amine überführt.

2. Verfahren nach Anspruch 1, dadurch gekennzeichnet, daß der aromatische Rest Phenyl, Naphthyl, Heteroaryl, der gegebenenfalls durch eine beliebige Anzahl Chlor, Fluor oder Bromatome, Trifluormethyl-, Nitro-, Cyano-, Carboxyl-, (C₁-C₄)-Alkoxycarbonyl-, (C₁-C₄)-Alkyl- oder (C₁-C₄)-Alkoxygruppen substituiert ist, bedeutet.

3. Verfahren nach Anspruche 1 oder 2, dadurch gekennzeichnet, daß man die Amide mit Chlor, Brom, Natriumhypochlorit oder Natriumhypobromit in Mengen von 1 bis 5 Mol, insbesondere 1,01 bis 2 Mol, bevorzugt 1,02 bis 1,2 Mol pro Mol Amid, in wäßrig alkalischen Medien umsetzt.

4. Verfahren nach Anspruch 3, dadurch gekennzeichnet, daß man aktives Chlor in Mengen von 30 bis 250, insbesondere von 100 bis 160 g, aktives Brom in Mengen von 60 bis 550, insbesondere von 200 bis 350 g pro kg wäßrige Lösung verwendet.

5. Verfahren nach mindestens einem der Ansprüche 1 bis 4, dadurch gekennzeichnet, daß man die Umsetzung der Amide bei Temperaturen von 0°C bis 50°C, insbesondere 10°C bis 40°C, durchführt.

6. Verfahren nach mindestens einem der Ansprüche 1 bis 5, dadurch gekennzeichnet, daß man (C₁-C₈)-Alkanole, die durch ein bis drei (C₁-C₄)-Alkoxygruppen, Fluor, Chlor, Bromatome, Nitrogruppen, Cyanogruppen, Trifluormethylgruppen oder (C₁-C₄)-Alkoxycarbonylgruppen substituiert sein können, insbesondere primäre Alkohole, bevorzugt Methanol und Ethanol, einsetzt.

7. Verfahren nach mindestens einem der Ansprüche 1 bis 5, dadurch gekennzeichnet, daß man Benzylalkohole, wobei der Phenylrest durch eine bis drei (C₁-C₄)-Alkylgruppen, (C₁-C₄)-Alkoxygruppen, Fluor, Chlor oder Bromatome, Nitrogruppen, Cyanogruppen, Trifluormethylgruppen, (C₁-C₄)-Alkoxycarbonylgruppen substituiert sein kann, einsetzt.

8. Verfahren nach mindestens einem der Ansprüche 1 bis 7, dadurch gekennzeichnet, daß man Alkohole in Mengen von 1 bis 20 Mol, insbesondere 1,1 bis 10 Mol, bevorzugt 1,05 bis 5 Mol pro Mol Amid einsetzt.

9. Verfahren nach mindestens einem der Ansprüche 1 bis 8, dadurch gekennzeichnet, daß man wäßrige Lösungen oder Suspensionen von Alkali- oder Erdalkalimetallhydroxiden, -carbonaten, -hydrogencarbonaten, -phosphaten,-hydrogenphosphaten, -dihydrogenphosphaten, -oxiden oder Mischungen dieser Verbindungen, insbesondere Natrium- oder Kaliumhydroxid verwendet.

10. Verfahren nach mindestens einem der Ansprüche 1 bis 9, dadurch gekennzeichnet, daß 2,6-Difluorbenzamid zu 2,6-Difluoranilin umgesetzt wird.

## Claims

1. A process for preparing aromatic amines by reacting aromatic amides with halogens or hypohalites in aqueous-alkaline solutions and/or suspensions by a Hofmann degradation reaction, which comprises carrying out the reaction in the presence of (C₁-C₈)-alkanols or benzyl alcohols at from -15 to 80°C and converting the reaction products into amines by hydrolysis, hydrogenation or reductive methods.

2. The process as claimed in claim 1, wherein the aromatic radical is phenyl, naphthyl or heteroaryl which is optionally substituted by an arbitrary number of chlorine, fluorine or bromine atoms, or trifluoromethyl, nitro, cyano, carboxyl, (C₁-C₄)-alkoxycarbonyl, (C₁-C₄)-alkyl or (C₁-C₄)-alkoxy groups.

3. The process as claimed in claim 1 or 2, wherein the amides are reacted in aqueous-alkaline media with chlorine, bromine, sodium hypochlorite or sodium hypobromite in quantities of from 1 to 5 mol, in particular of from 1.01 to 2 mol, preferably of from 1.02 to 1.2 mol, per mole of amide.

4. The process as claimed in claim 3, wherein active chlorine is used in quantities of from 30 to 250, in particular of from 100 to 160 g, and active bromine in quantities of from 60 to 550, in particular of from 200 to 350 g, per kg of aqueous solution.

5. The process as claimed in at least one of claims 1 to 4, wherein the amides are reacted at temperatures of from 0°C to 50°C, in particular of from 10°C to 40°C.

6. The process as claimed in at least one of claims 1 to 5, wherein (C₁-C₈)-alkanols, which can be substituted by one to three (C₁-C₄)-alkoxy groups, fluorine, chlorine, bromine atoms, nitro groups, cyano groups, trifluoromethyl groups or (C₁-C₄)-alkoxycarbonyl groups, are employed, in particular primary alcohols, preferably methanol and ethanol.

7. The process as claimed in at least one of claims 1 to 5, wherein benzyl alcohols, whose phenyl radical can be substituted by one to three (C₁-C₄)-alkyl groups, (C₁-C₄)-alkoxy groups, fluorine, chlorine or bromine atoms, nitro groups, cyano groups, trifluoromethyl groups or (C₁-C₄)-alkoxycarbonyl groups, are employed.

8. The process as claimed in at least one of claims 1 to 7, wherein alcohols are employed in quantities of from 1 to 20 mol, in particular of from 1.1 to 10 mol, preferably of from 1.05 to 5 mol, per mole of amide.

9. The process as claimed in at least one of claims 1 to 8, wherein aqueous solutions or suspensions of alkali metal or alkaline earth metal hydroxides, carbonates, hydrogen carbonates, phosphates, hydrogen phosphates, dihydrogen phosphates or oxides, or mixtures of these compounds, in particular sodium hydroxide or potassium hydroxide, are used.

10. The process as claimed in at least one of claims 1 to 9, wherein 2,6-difluorobenzamide is converted into 2,6-difluoroaniline.

## Revendications

1. Procédé pour la préparation d'amines aromatiques par réaction d'amides aromatiques en solutions et/ou suspensions hydroalcalines avec des halogènes ou des hypohalogénites selon la réaction de dégradation d'Hofmann, caractérisé en ce qu'on effectue la réaction en présence d'alcanols en C₁-C₈ ou d'alcools benzyliques à une température de -15 à 80°C, et en ce que l'on transforme les produits réactionnels en amines par hydrolyse, hydrogénation ou des méthodes réductrices.

2. Procédé selon la revendication 1, caractérisé en ce que le radical aromatique représente phényle, naphtyle, hétéroaryle, qui est éventuellement substitué par un nombre quelconque d'atomes de chlore, fluor ou brome, de groupes trifluorométhyle, nitro, cyano, carboxyle, alcoxy-(en C₁-C₄)-carbonyle, alkyle en C₁-C₄ ou alcoxy en C₁-C₄.

3. Procédé selon la revendication 1 ou 2, caractérisé en ce qu'on fait réagir les amides dans des milieux hydroalcalins avec du chlore, du brome, de l'hypochlorite de sodium ou de l'hypobromite de sodium, en des quantités de 1 à 5 mol, en particulier de 1,01 à 2 mol, de préférence de 1,02 à 1,2 mol par mol d'amide.

4. Procédé selon la revendication 3, caractérisé en ce qu'on utilise du chlore actif en des quantités de 30 à 250, en particulier de 100 à 160 g, du brome actif en des quantités de 60 à 550, en particulier de 200 à 350 g par kg de solution aqueuse.

5. Procédé selon au moins l'une des revendications 1 à 4, caractérisé en ce qu'on effectue la réaction des amides à des températures de 0°C à 50°C, en particulier de 10°C à 40°C.

6. Procédé selon au moins l'une des revendications 1 à 5, caractérisé en ce que l'on utilise des alcanols en C₁-C₈, qui peuvent être substitués par 1 à 3 groupes alcoxy en C₁-C₄, des atomes de fluor, chlore, brome, des groupes nitro, des groupes cyano, des groupes trifluorométhyle ou des groupes alcoxy-(en C₁-C₄)-carbonyle, en particulier des alcools primaires, de préférence le méthanol et l'éthanol.

7. Procédé selon au moins l'une des revendications 1 à 5, caractérisé en ce que l'on utilise des alcools benzyliques, dans lesquels le radical phényle peut être substitué par 1 à 3 groupes alkyle en C₁-C₄, groupes alcoxy en C₁-C₄, atomes de fluor, chlore ou brome, groupes nitro, groupes cyano, groupes trifluorométhyle, groupes alcoxy-(en C₁-C₄)-carbonyle.

8. Procédé selon au moins l'une des revendications 1 à 7, caractérisé en ce que l'on utilise des alcools en des quantités de 1 à 20 mol, en particulier de 1,1 à 10 mol, de préférence de 1,05 à 5 mol par mol d'amide.

9. Procédé selon au moins l'une des revendications 1 à 8, caractérisé en ce que l'on utilise des solutions ou des suspensions aqueuses d'hydroxydes, carbonates, carbonates acides, phosphates, phosphates acides, phosphates diacides, oxydes de métaux alcalins ou alcalino-terreux, ou des mélanges de ces composés, en particulier l'hydroxyde de sodium ou de potassium.

10. Procédé selon au moins l'une des revendications 1 à 9, caractérisé en ce que l'on transforme le 2,6-difluorobenzamide en 2,6-difluoroaniline.
